# EUROPÄISCHE PATENTSCHRIFT

(11) **EP 2 582 669 B1**
(45) Veröffentlichungstag und Bekanntmachung des Hinweises auf die Patenterteilung: **07.10.2015**
(21) Anmeldenummer: 11724663.7
(22) Anmeldetag: 14.06.2011
(51) Int. Cl.: C07D 211/02, C07D 241/04, C07D 295/023

(54) **VERFAHREN ZUR HERSTELLUNG EINES ZYKLISCHEN TERTIÄREN METHYLAMINS**
PROCESS FOR PREPARING A CYCLIC TERTIARY METHYLAMINE
PROCÉDÉ DE PRODUCTION D'UNE MÉTHYLAMINE TERTIAIRE CYCLIQUE

(30) Priorität: 15.06.2010 EP 10166017
(43) Veröffentlichungstag der Anmeldung: 24.04.2013
(73) Patentinhaber: BASF SE, 67056 Ludwigshafen (DE)
(72) Erfinder: WIGBERS, Christof Wilhelm, 68167 Mannheim (DE); MELDER, Johann-Peter, 67459 Böhl-Iggelheim (DE); STEIN, Bernd, 64665 Alsbach-Hähnlein (DE); MEIßNER, Harald, 67454 Haßloch (DE)
(86) Internationale Anmeldenummer: PCT/EP2011/059848
(87) Internationale Veröffentlichungsnummer: WO 2011/157710

(56) Entgegenhaltungen:
- EP-A1- 0 257 443
- EP-A1- 0 440 829
- EP-A1- 0 816 350
- WO-A1-2007/036496
- WO-A1-2008/006754
- WO-A2-2009/027249

## Beschreibung

Die vorliegende Erfindung betrifft ein Verfahren zur Herstellung eines zyklischen tertiären Methylamins der Formel I 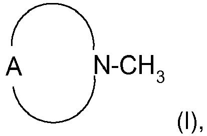 in der
A eine C₄-Alkylengruppe, eine C₅-Alkylengruppe oder eine -(CH₂)₂-B-(CH₂)₂-Gruppe bedeutet, wobei B Sauerstoff (O) oder einen Rest N-R¹ bedeutet und R¹ C₁- bis C₅-Alkyl, Aryl oder C₅- bis C₇-Cycloalkyl bedeutet.

Die tertiären Methylamine besitzen in der chemischen Industrie große Bedeutung für die Herstellung von Prozess-Chemikalien, Katalysatoren und Zwischenprodukten für höher veredelte Produkte.

Aus EP 257 443 A1 (BASF AG) ist bekannt, Trialkylamine (z. B. Dimethylethylamin) durch Umsetzung von Ammoniak oder primären Aminen mit primären einwertigen a-liphatischen Alkoholen mit 2 bis 24 Kohlenstoffatomen, bevorzugt in Gegenwart von Wasserstoff und in Gegenwart eines Hydrier-/Dehydrierkatalysators herzustellen. Die Reaktion wird kontinuierlich in flüssiger Phase bei einem Gesamtdruck von 50 bis 300 bar und einem molaren Überschuss von 1 bis 15 mol des Alkohols über den Ammoniak bzw. das primäre Amin durchgeführt. Außerdem erfolgt die Alkylierung in Gegenwart von Alkalimetall- und/oder Erdalkalimetalloxiden und/oder-hydroxiden. Der Hydrier-/Dehydrier-Katalysator enthält im wesentlichen Kupfer.

In den Beispielen 1 und 3 wird Ammoniak mit Ethanol bzw. n-Butanol in Gegenwart von Natronlauge zu Triethylamin bzw. Tri-n-butylamin umgesetzt. Gearbeitet wird bei 230 bis 250 °C, Gesamtdrucken von 200 bar und Wasserstoffpartialdrucken von > 150 bar bzw. 120 bar. Es werden neben den tertiären Aminen als Zielprodukten nur geringe Mengen an sekundären Aminen gebildet.

In Beispiel 2 wird anstelle von Ammoniak das primäre Amin n-Pentylamin eingesetzt, das in Gegenwart von Natronlauge kontinuierlich mit n-Pentanol zu Tri-n-pentylamin umgesetzt wird. Das Molverhältnis von n-Pentylamin zu n-Pentanol liegt bei 1 zu 4. Die Reaktionstemperatur beträgt 230 - 235 °C, der Gesamtdruck 60 bar, wovon rund 10 bar auf den Wasserstoffpartialdruck entfallen. Das Molverhältnis von Tri-n-pentylamin zu Di-n-pentylamin im Reaktionsaustrag betrug 1 zu 0,1. Überschüssiges n-Pentanol wurde nicht in die Synthesestufe zurückgeführt.

Nach Anspruch 4 werden C₁-C₂₄-Alkohole in reiner Form oder in Form ihrer Mischungen eingesetzt. Ausführungsbeispiele unter Verwendung von Methanol sind in EP 257 443 A1 nicht enthalten.

In ähnlicher Weise wird nach EP 227 904 A1 (BASF AG) Dimethylamin mit Ethanol bzw. n-Butanol zu Dimethylethylamin bzw. Dimethyl-n-butylamin in der Flüssigphase umgesetzt. Die Umsetzung erfolgt in Gegenwart von Alkalimetallhydroxid und eines Katalysators, der als aktives Metall im wesentlichen nur Kupfer enthält bzw. ein reiner Kupferkatalysator ist. Verwendbar sind einwertige aliphatische Alkohole mit 2 bis 4 Kohlenstoffatomen. Methanol wird in EP 227 904 nicht genannt.

Nachteilig beim Arbeiten gemäß EP 257 443 A1 und EP 227 904 A1 ist, dass die Alkylierung bevorzugt in Gegenwart von Wasserstoff durchgeführt wird. In allen Ausführungsbeispielen wird in Gegenwart von Wasserstoff gearbeitet. Für diese Ausführungsweise sind spezielle Sicherheitsmaßnahmen einzuhalten. Hinzu kommt, dass die dem Reaktionsgemisch zugesetzten Alkalimetall- und/oder Erdalkalimetalloxide und Alkalimetall- bzw. Erdalkalimetallhydroxide nach der Alkylierungsreaktion aus dem Reaktionsaustrag wieder abgetrennt werden müssen. Außerdem kann beim Arbeiten mit Methanol und dessen Dehydrierung zu Formaldehyd die Cannizarro-Reaktion ablaufen, die in Gegenwart der basischen Alkalimetallsalze Methanol und Alkalimetallformiat liefert (Beyer/Walther, Lehrbuch der organischen Chemie, 24. Auflage, 2004, S. Hirzel-Verlag, Seite 215).

Dass weder in EP 257 443 noch in EP 227 904 Ausführungsbeispiele mit Methanol als Alkylierungsmittel zu finden sind, ist wohl auf die potentielle starke Nebenproduktbildung durch die Cannizzaro-Reaktion zurückzuführen.

US 4,910,304 A (BASF AG) offenbart die Herstellung von N-Methylpiperidin und N-Methylmorpholin durch Reaktion von Pentandiol bzw. Diethylenglykol (DEG) mit Methylamin und 45 %iger wässriger KOH-Lösung an einem Cu/Al-Vollkontakt bei 245°C und 250 bar.

EP 137 478 A (BASF AG) betrifft Verfahren zur Herstellung von N-Methylpiperidin oder N-Methylmorpholin durch katalytische Aminierung von Pentandiol mit Methylamin in der Gasphase zwischen 5 und 25 bar an einem kupferhaltigen Katalysator, der durch Temperung eines basischen Kupfer und Aluminium enthaltenden Carbonats erhalten wurde.

EP 235 651 A1 (BASF AG) lehrt ein Verfahren zur Herstellung von N-Methylpiperazin aus Diethanolamin und Methylamin an metallhaltigen Katalysatoren. Die Umsetzung wird in der Flüssigphase (Rieselfahrweise) durchgeführt (Seite 3, letzter Absatz). Gemäß Beispiel wird ein Cu/Al₂O₃-Katalysator eingesetzt.

EP 816 350 A1 (BASF AG) beschreibt Verfahren zur Herstellung von N-Methylpiperidin und N-Methylmorpholin durch Umsetzung von primärem Amin mit einem Diol an einem Kupferkatalysator, welcher durch Tränkung von SiO₂-Kugeln mit basischem Kupfercarbonat erhalten wurde, in der Flüssig- oder Gasphase.

US 4,739,051 A (BASF AG) lehrt die Herstellung von Morpholin und Piperidin durch Reaktion von DEG oder Pentandiol mit Ammoniak unter Hydrierbedingungen in der Gasphase bei Normaldruck und 200 °C an einem Cu/Ni/Al-Vollkatalysator.

EP 514 692 A2 (BASF AG) offenbart Verfahren zur Herstellung von Aminen aus Alkoholen in Gegenwart Kupfer und Nickel und Zirkonium- und/oder Aluminiumoxid enthaltender Katalysatoren.

EP 440 829 A1 (US 4,910,304) (BASF AG) beschreibt die Aminierung von Diolen an Kupferkatalysatoren. Die Umsetzung wird in der Flüssigphase (Rieselfahrweise) durchgeführt (Seite 3, letzter Absatz). Geeignete Katalysatoren sind die in DE 24 45 303 A (BASF AG) offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird.

WO 07/036496 A1 (BASF AG) beschreibt die Umsetzung von Diethylenglykol mit Ammoniak in Gegenwart von Cu-Ni-Co - Katalysatoren.

WO 05/110969 A1 (BASF AG) beschreibt ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupferhaltigen Katalysators, wobei die katalytisch aktive Masse des Katalysators vor dessen Reduktion mit Wasserstoff 20 bis 85 Gew.-% Aluminiumoxid (Al₂O₃), Zirkoniumdioxid (ZrO₂), Titandioxid (TiO₂) und/oder Siliziumdioxid (SiO₂) enthält und die Umsetzung in der Gasphase isotherm in einem Rohrreaktor erfolgt.

WO 2010/031719 A1 (BASF SE) betrifft ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300 °C in Gegenwart eines kupfer- und aluminiumoxidhaltigen Katalysators, wobei die Umsetzung in der Gasphase erfolgt und die katalytisch aktive Masse des Katalysators vor dessen Reduktion mit Wasserstoff Aluminiumoxid und sauerstoffhaltige Verbindungen des Kupfers enthält und der Katalysatorformkörper spezifiziert ist.

WO 2008/006754 A1 (BASF AG) beschreibt ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart eines zirkoniumdioxid- und nickelhaltigen Katalysators, wobei die katalytisch aktive Masse des Katalysators vor dessen Reduktion mit Wasserstoff sauerstoffhaltige Verbindungen des Zirkoniums, Kupfers und Nickels und im Bereich von 0,5 bis 6 Gew.-% sauerstoffhaltige Verbindungen des Silbers, berechnet als AgO, enthält,

Der vorliegenden Erfindung lag die Aufgabe zugrunde, Nachteilen des Stands der Technik abzuhelfen und ein verbessertes wirtschaftliches Verfahren zur Herstellung eines zyklischen tertiären Methylamins aufzufinden. Insbesondere soll das Verfahren bessere Ausbeuten, Raum-Zeit-Ausbeuten (RZA) und Selektivitäten ermöglichen, im Besonderen auch ohne den Zusatz von Wasserstoff. [Raum-Zeit-Ausbeuten werden angegeben in ,Produktmenge / (Katalysatorvolumen ● Zeit)' (kg/(I_{Kat.} ● h)) und/oder ,Produktmenge / (Reaktorvolumen ● Zeit)' (kg/(I_{Reaktor} ● h)]. Demgemäß wurde ein Verfahren zur Herstellung eines zyklischen tertiären Methylamins der Formel I 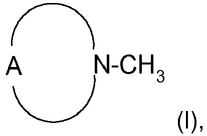 in der
A eine C₄-Alkylengruppe, eine C₅-Alkylengruppe oder eine -(CH₂)₂-B-(CH₂)₂-Gruppe bedeutet, wobei B Sauerstoff (O) oder einen Rest N-R¹ bedeutet und R¹ C₁- bis C₅-Alkyl, Aryl oder C₅- bis C₇-Cycloalkyl bedeutet,
gefunden, welches dadurch gekennzeichnet ist, dass man
(i) einen Aminoalkohol II aus der Gruppe 1,4-Aminobutanol, 1,5-Aminopentanol, Aminodiglykol (ADG) bzw. Aminoethyl-ethanolamin der Formel IIa 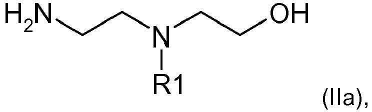 in der R¹ wie oben genannnt oder auch Wasserstoff (H) bedeutet, wobei im Amin I dann R¹ = CH₃ ist,
   mit Methanol in einem Reaktor bei einer Temperatur im Bereich von 150 bis 270 °C in Gegenwart eines kupferhaltigen Heterogenkatalysators in der Flüssigphase umsetzt, wobei das Methanol in der 1- bis 25-fachen molaren Menge bezogen auf den eingesetzten Aminoalkohol (II, IIa) eingesetzt wird.

Bevorzugt ist, dass man in einem nachfolgenden Schritt (ii) aus dem Reaktionsaustrag der Umsetzung (Reaktionsschritt (i)) in einer ersten Destillationseinheit unumgesetztes Methanol als Kopfprodukt abtrennt und in den Reaktionsschritt (i) zurückführt, wobei der Kohlendioxidgehalt des zurückgeführten Methanols soweit abgesenkt wird, dass in der Methanol-Zuführungsleitung zum Reaktor und der Eintrittsstelle des Methanols in den Reaktor keine Feststoffbildung erfolgt.

Weiter bevorzugt ist, dass man in einem nachfolgenden Schritt (iii) aus dem Sumpfprodukt der ersten Destillationseinheit durch Extraktion mit wässriger Alkali- oder Erdalkalihydroxid-Lösung oder durch Azeotropdestillation mit einem Kohlenwasserstoff in einer zweiten Destillationseinheit Reaktionswasser entfernt, und (iv) den resultierenden Austrag zur Gewinnung von tertiärem Amin I fraktionierend destilliert.

Das erfindungsgemäße Verfahren ermöglicht es, die Teilschritte der zyklischen Methylierung, nämlich Zyklisierung und Methylierung, in einem Reaktionsschritt durchzuführen. Dabei werden die Aminoalkohole II, IIa mit hohen Umsätzen und Ausbeuten zum zyklischen tertiären Methylamin I umgesetzt. Eine Rückführung von unvollständig methylierten oder zyklisierten Zwischenprodukten ist daher im Allgemeinen nicht notwendig.

Die Reaktionstemperatur für die Herstellung des tertiären Amins I beträgt 150 bis 270 °C, bevorzugt 170 bis 250 °C, besonders bevorzugt 180 bis 230 °C.

Nach der Stöchiometrie der Alkylierung mit Methanol muss kein Wasserstoff zugeführt werden. Demgemäß wird in einer Verfahrensausgestaltung im Reaktionsschritt (i) dem Reaktor kein Wasserstoff (H₂) zugeführt.

Um die Aktivität des Hydrierkatalysators über sehr lange Zeiten konstant zu halten, kann es jedoch vorteilhaft sein, dem Reaktionsgemisch kontinuierlich oder von Zeit zu Zeit Wasserstoff zuzuführen.

Der Reaktionsdruck im Reaktor setzt sich bei der jeweiligen Reaktionstemperatur aus den Partialdrücken der Einsatzstoffe und der Reaktionsprodukte, gegebenenfalls von Lösungsmittel und zugeführtem Wasserstoff zusammen. Durch Aufpressen von Wasserstoff wird der Druck auf den gewünschten Reaktionsdruck erhöht.

Der Gesamtdruck (absolut) beträgt bevorzugt 50 bis 150 bar, bevorzugt 60 bis 130 bar, besonders bevorzugt 70 bis 120 bar.

Falls Wasserstoff eingesetzt wird, beträgt der Wasserstoffpartialdruck, der sich aus zugefügtem und in situ generiertem H₂ ergibt, dabei besonders 0,01 bis 130 bar, bevorzugt 0,1 bis 100 bar, besonders bevorzugt 1 bis 80 bar.

Wird die Alkylierung kontinuierlich durchgeführt, so beträgt die Raum-Zeit-Ausbeute besonders 0,01 bis 5 kg/(I_{Kat}. ● h), bevorzugt 0,05 bis 3 kg/(I_{Kat.} ● h), besonders bevorzugt 0,1 bis 1,0 kg/(I_{Kat.}● h).

Methanol wird bevorzugt in deutlichem Überschuss zu dem Aminoalkohol (II, IIa) eingesetzt und kann als Reaktionspartner und gleichzeitig als Lösungsmittel dienen. Das Molverhältnis von Aminoalkohol zu Methanol beträgt 1 zu 25, bevorzugt 2 zu 20, besonders bevorzugt 3 zu 15. Ein hohes Molverhältnis kann sich positiv auf die Selektivität der tertiären Amine I auswirken, sofern der Methanolüberschuss die intramolekulare Zyklisierung nicht beeinträchtigt. Zudem können in einem Überschuss Methanol eventuell entstehende Carbamate gelöst werden.

Es kann aber auch vorteilhaft sein, zusätzlich ein unter den Reaktionsbedingungen inertes Lösungsmittel mit zu verwenden. Hierfür kommen aliphatische, cycloaliphatische oder aromatische Lösungsmittel in Frage. Beispiele hierfür sind n-Hexan, n-Octan, Cyclohexan, Methylcyclohexan, Toluol, o-, m- oder p-Xylol oder Gemische dieser Verbindungen.

Das Gemisch aus Aminoalkohol, Methanol und inertem Lösungsmittel kann 20 bis 70 Gew.-%, bevorzugt 30 bis 60 Gew.-%, an inertem Lösungsmittel enthalten.

In einer speziellen Ausführung erfolgt die Umsetzung in mehreren (z. B. 2, 3, 4, 5, etc.) Reaktoren. Bevorzugt ist eine Kombination aus zwei Reaktoren. Dabei können gleiche oder verschiedene Reaktoren eingesetzt werden. Die Reaktoren können untereinander beliebig verschaltet sein, z. B. parallel oder in Reihe. In einer bevorzugten Ausführung werden zwei in Reihe geschaltete Reaktoren eingesetzt. Werden mehrere Reaktoren eingesetzt, so können diese gleiche oder verschiedene Temperaturen aufweisen. Vorzugsweise ist die Temperatur im n. (n-ten) Reaktor um wenigstens 10 °C, besonders bevorzugt um wenigstens 20 °C, insbesondere um wenigstens 30 °C höher als die Temperatur im (n - 1.) (n minus ersten) Reaktor. Der Reaktionsdruck kann beim Einsatz mehrerer Reaktoren in den einzelnen Reaktoren gleich oder verschieden sein. In einer speziellen Ausführungsform enthält dabei nur ein Teil der Reaktoren Katalysator. So kann z. B. eine Kombination aus zwei Reaktoren eingesetzt werden, von denen nur einer Katalysator enthält. In dieser Variante kann das Reaktionsgemisch zunächst in einem Reaktor ohne Katalysator vorerwärmt und dann anschließend zur Reaktion in einen Reaktor mit Katalysator überführt werden. Zum Überführen des Reaktionsgemisches kann z. B. ein Inertgas, wie Stickstoff und Edelgase oder Wasserstoff, eingesetzt werden, mit dem das Gemisch vom einen in einen anderen Reaktor gedrückt wird. Zusätzlich kann das Gas auch zur Einstellung des gewünschten Reaktionsdrucks dienen.

Als Katalysatoren eignen sich prinzipiell Hydrierkatalysatoren, bevorzugt kupferhaltige, heterogene Katalysatoren.

Prinzipiell eignet sich eine Vielzahl von kupferhaltigen Katalysatoren, die zusätzlich wenigstens ein weiteres Element der I., II., III., IV. oder V. Hauptgruppe, der I., II., IV., V., VII. oder VIII. Nebengruppe sowie der Lanthanide enthalten können (IUPAC: Gruppen 1 bis 15 und die Lanthanide), insbesondere Ca, Mg, Al, La, Ti, Zr, Cr, Mo, W, Mn, Ni, Co, Zn und Kombinationen davon.

Eine spezielle Ausführungsform von vorteilhaften Katalysatoren sind Raney-Katalysatoren, speziell Raney-Kupfer sowie kupferhaltige Metalllegierungen in Form eines Raney-Katalysators. Bevorzugt sind Raney-Katalysatoren, deren Metallkomponente zu wenigstens 95 Gew.-%, insbesondere zu wenigstens 99 Gew.-%, aus Kupfer besteht. Raney-Kupfer kann in an sich bekannter Weise durch Behandeln von Kupfer-Aluminium-Legierungen mit Alkalihydroxiden hergestellt werden.

Eine weitere spezielle Ausführungsform von Katalysatoren, die sich besonders vorteilhaft für einen Einsatz eignen, sind Katalysatoren, die Kupfer in oxidischer Form sowie gegebenenfalls zusätzlich in elementarer Form enthalten.

Geeignet sind z. B. Katalysatoren, die auf einem Träger aus Kieselsäure Nickel und Kupfer neben anderen Metallen als aktive Bestandteile enthalten. Solche Katalysatoren werden z. B. in DE 26 28 087 A beschrieben. Die aktive Masse dieser Katalysatoren enthält speziell 40 bis 80 Gew.-% Nickel, 10 bis 50 Gew.-% Kupfer und 2 bis 10 Gew.-% Mangan. EP 434 062 A beschreibt Hydrierkatalysatoren, die durch Reduktion eines Precursors aus Oxiden des Kupfers, Aluminiums und wenigstens eines weiteren Metalls, ausgewählt unter Magnesium, Zink, Titan, Zirkon, Zinn, Nickel und Kobalt, erhältlich sind. Geeignet sind auch die in der DE 102 18 849 A beschriebenen Hydrierkatalysatoren, die 0,1 bis 10 Gew.-% Chrom, berechnet als Cr₂O₃, 0,1 bis 10 Gew.-% Calcium, berechnet als CaOx, und 5 bis 20 Gew.-% Kupfer, berechnet als CuO, abgeschieden auf einem Siliciumdioxid-Trägermaterial und jeweils bezogen auf das Gesamtgewicht des calcinierten Katalysators, enthalten. Aus der DE 40 21 230 A sind Kupfer-Zirkonoxid-Katalysatoren bekannt, wobei das Verhältnis von Kupferatomen zu Zirkoniumatomen, ausgedrückt als Gewichtsverhältnis, 1 : 9 bis 9 : 1 beträgt. DE 4 028 295 A beschreibt geeignete Kupfer-Mangan-Hydrierkatalysatoren. EP 552 463 A beschreibt Katalysatoren, wobei die oxidischer Form im Wesentlichen der Zusammensetzung CuaAl_{b}Zr_{c}Mn_{d}Oₓ entspricht, wobei die folgenden Beziehungen gelten: a > 0; b > 0; c ≥ 0; d > 0; a > b/2; b > a/4; a > c; a > d; und x die zur Wahrung der Elektroneutralität pro Formeleinheit erforderliche Anzahl von Sauerstoffionen bezeichnet. EP 552 463 A beschreibt auch Katalysatoren mit einem geringeren Anteil an Aluminiumoxid. Der Katalysator nach dieser Ausführungsform entspricht im Wesentlichen der Zusammensetzung CuₐAl_{b}Zr_{c}Mn_{d}Oₓ, wobei die folgenden Beziehungen gelten: a > 0; a/40 ≤ b ≤ a/4; c ≥ 0; d > 0; a > c; 0,5d ≤ a ≤ 0,95d und x, die Wahrung der Elektroneutralität pro Formeleinheit erforderliche Anzahl von Sauerstoffionen bezeichnet. WO 2006/005505 A beschreibt Katalysatorformkörper, die sich für den Einsatz in dem erfindungsgemäßen Verfahren besonders eignen. In einer bevorzugten Ausführung umfasst das oxidische Katalysatormaterial
(a) Kupferoxid mit einem Anteil im Bereich von 50 ≤ x ≤ 80 Gew.-%, bevorzugt 55 ≤ x ≤ 75 Gew.-%,
(b) Aluminiumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkonsiums, bevorzugt des Lanthans und/oder Wolframs, mit einem Anteil im Bereich von 2 ≤ z ≤ 20 Gew.-%, bevorzugt 3 ≤ z ≤ 15 Gew.-%, jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugte Katalysatoren umfassen die folgenden Metalle in oxidischer Form, reduzierter Form (elementarer Form) oder einer Kombination davon. Metalle, die in mehr als einer Oxidationsstufe stabil sind, können vollständig in einer der Oxidationsstufen oder in verschiedenen Oxidationsstufen eingesetzt werden:
- Cu
- Cu, Ti
- Cu, Zr
- Cu, Mn
- Cu, Al
- Cu, Ni, Mn
- Cu, Al, wenigstens ein weiteres Metall, ausgewählt unter La, W, Mo, Mn, Zn, Ti, Zr, Sn, Ni, Co
- Cu, Zn, Zr
- Cu, Cr, Ca
- Cu, Cr, C
- Cu, Al, Mn, gegebenenfalls Zr.

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid und Siliciumdioxid.

In der bevorzugten Ausführungsform des erfindungsgemäßen Verfahrens verwendet man Kupferkatalysatoren, die in der DE 2 445 303 A1 (BASF AG) beschrieben worden sind. Sie können als amorphe Produkte der thermischen Zersetzung und Reduktion basischer Kupfer-Aluminium-Carbonate angesehen und dadurch erhalten werden, dass man verdünnte oder mäßig konzentrierte, zweckmäßig unter 3-molare Lösungen von Kupfer- und Aluminiumsalzen mit Alkalicarbonat bei pH 8 - 10 fällt und die erhaltenen Niederschläge vor oder nach entsprechender Formung bei einer Temperatur von 350 - 600 °C zersetzt. Nach üblicher Reduzierung, bevorzugt in Gegenwart des bei der späteren Umsetzung verwendeten Alkohols, erhält man hochaktive, für das vorliegende Verfahren bestens geeignete Katalysatoren.

Bei der nach dem erfindungsgemäßen Verfahren ebenfalls möglichen Suspensionsfahrweise ist der reduzierte Kupferkatalysator in den Reaktionskomponenten Alkohol und Amine suspendiert. Geeignete Katalysatoren sind z. B. Raney-Kupfer oder die oben beschriebenen Kupferkatalysatoren in gepulverter Form. Bevorzugt ist jedoch ein Kupfermaterial, das man dadurch erhalt, dass man Kupferformiat in Gegenwart eines Alkohols und Dialkylamin auf 200 - 250 °C erhitzt. Die Bildungsweise eines solchen Katalysators wird z. B. in der EP 70 512 A beschrieben.

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Im erfindungsgemäßen Verfahren werden die Katalysatoren besonders bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang wird oxidisches Trägermaterial, besonders bevorzugt Aluminiumoxid (Al₂O₃), als zur katalytisch aktiven Masse gehörig gewertet.

Die Katalysatoren werden bevorzugt dergestalt eingesetzt, dass man die katalytisch aktive Masse nach Mahlung, Vermischung mit Formhilfsmitteln, Formung und Temperung als Katalysatorformkörper-z.B. als Tabletten, Kugeln, Ringe, Extrudate (z.B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und des o. g. Katalysatorträgermaterials definiert und enthält bevorzugt im wesentlichen die folgenden Bestandteile:
Aluminiumoxid (Al₂O₃) und sauerstoffhaltige Verbindungen des Kupfers und bevorzugt sauerstoffhaltige Verbindungen des Natriums.

Die Summe der o. g. Bestandteile der katalytisch aktiven Masse, berechnet als Al₂O₃, CuO und Na₂O, beträgt üblicherweise 70 bis 100 Gew.-%, bevorzugt 80 bis 100 Gew.-%, besonders bevorzugt 90 bis 100 Gew.-%, weiter bevorzugt 98 bis 100 Gew.-%, weiter bevorzugt ≥ 99 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Ni bzw. NiO, Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie K₂O; Alkalimetallcarbonate, wie Na₂CO₃; Erdalkalimetalloxide, wie CaO, SrO; Erdalkalimetallcarbonate, wie MgCO₃, CaCO₃ und BaCO₃; Boroxid (B₂O₃).

Die katalytisch aktive Masse der im erfindungsgemäßen Verfahren eingesetzten Katalysatoren enthält nach deren letzter Wärmebehandlung und vor deren Reduktion mit Wasserstoff besonders
25 bis 80 Gew.-%, bevorzugt 30 bis 70 Gew.-%, besonders bevorzugt 35 bis 60 Gew.-%, Aluminiumoxid (Al₂O₃) und
20 bis 75 Gew.-%, bevorzugt 30 bis 70 Gew.-%, besonders bevorzugt 40 bis 65 Gew.-%, ganz besonders bevorzugt 45 bis 60 Gew.-%, sauerstoffhaltige Verbindungen des Kupfers, berechnet als CuO,
0 bis 2 Gew.-%, bevorzugt 0,05 bis 1 Gew.-%, besonders bevorzugt 0,1 bis 0,5 Gew.-%, sauerstoffhaltige Verbindungen des Natriums, berechnet als Na₂O,
weniger als 5 Gew.-%, z. B. 0,1 bis 4 Gew.-%, bevorzugt weniger als 1 Gew.-%, z. B. 0 bis 0,8 Gew.-%, sauerstoffhaltige Verbindungen des Nickels, berechnet als NiO.

Die katalytisch aktive Masse des Katalysators enthält vor dessen Reduktion mit Wasserstoff besonders bevorzugt weniger als 1 Gew.-%, z. B. 0 bis 0,5 Gew.-%, sauerstoffhaltige Verbindungen des Kobalts, berechnet als CoO.

Ganz besonders bevorzugt enthält die katalytisch aktive Masse des im erfindungsgemäßen Verfahren eingesetzten Katalysators kein Nickel, kein Kobalt und/oder kein Ruthenium, jeweils weder in metallischer (Oxidationsstufe 0) noch in einer ionischen, insb. oxidierten, Form.

Bei den sauerstoffhaltigen Verbindungen des Kupfers handelt es sich insbesondere um Kupfer-(I)-oxid und Kupfer-(II)-oxid, bevorzugt um Kupfer-(II)-oxid.

Ganz besonders bevorzugt enthält die katalytisch aktive Masse des im erfindungsgemäßen Verfahren eingesetzten Katalysators kein Zirkoniumdioxid (ZrO₂), Titandioxid (TiO₂) und/oder Siliziumdioxid (SiO₂).

In einer besonders bevorzugten Ausführungsform enthält die katalytisch aktive Masse der im erfindungsgemäßen Verfahren eingesetzten Katalysatoren keine weitere katalytisch aktive Komponente, weder in elementarer noch in ionischer Form.

In der besonders bevorzugten Ausführungsform ist die katalytisch aktive Masse nicht mit weiteren Metallen oder Metallverbindungen dotiert.

Bevorzugt sind jedoch aus der Metallgewinnung von Cu, ggf. Ni, herrührende übliche Begleit-Spurenelemente hiervon ausgenommen.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten Aluminium, Kupfer, ggf. Natrium mit Wasser und nachfolgendes Extrudieren und Tempern der so erhaltenen Masse erhältlich.

Die im erfindungsgemäßen Verfahren bevorzugt verwendeten Katalysatoren können auch durch Tränkung von Aluminiumoxid (Al₂O₃), das beispielsweise in Form von Pulver oder Tabletten-Formkörpern vorliegt, hergestellt werden.

Aluminiumoxid kann dabei in verschiedenen Modifikationen eingesetzt werden, bevorzugt sind α- (alpha), γ- (gamma) oder θ-Al₂O₃ (theta-Al₂O₃). Besonders bevorzugt wird γ-Al₂O₃ eingesetzt.

Die Herstellung von Formkörpern des Aluminiumoxids kann nach den üblichen Verfahren erfolgen.

Die Tränkung des Aluminiumoxids erfolgt ebenfalls nach den üblichen Verfahren, wie z. B. in EP 599 180 A, EP 673 918 A oder A. B. Stiles, Catalyst Manufacture-Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. kalziniert.

Die Tränkung kann nach der sogenannten "incipient wetness"-Methode erfolgen, bei der das anorganische Oxid (d. h. Aluminiumoxid) entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das anorganische Oxid mit einer größeren Metallmenge beaufschlagt werden soll.

Zur Aufbringung mehrerer Metallkomponenten auf das anorganische Oxid kann die Tränkung gleichzeitig mit ggf. allen Metallsalzen oder in beliebiger Reihenfolge der ggf. einzelnen Metallsalze nacheinander erfolgen.

Bevorzugt werden zur Herstellung der im erfindungsgemäßen Verfahren bevorzugt verwendeten Katalysatoren Fällungsmethoden angewandt. So können sie beispielsweise durch eine gemeinsame Fällung der Komponenten aus einer wässrigen Salzlösung mittels Mineralbasen in Gegenwart einer Aufschlämmung einer schwerlöslichen, sauerstoffhaltigen Aluminium-Verbindung und anschließendes Waschen, Trocknen und Calcinieren des erhaltenen Präzipitats erhalten werden. Als schwerlösliche, sauerstoffhaltige Aluminiumverbindung kann beispielsweise Aluminiumoxid Verwendung finden. Die Aufschlämmungen der schwerlöslichen Aluminiumverbindung kann durch Suspendieren feinkörniger Pulver dieser Verbindung in Wasser unter kräftigem Rühren hergestellt werden. Vorteilhaft werden diese Aufschlämmungen durch Ausfällen der schwerlöslichen Aluminiumverbindung aus wässrigen Aluminium-Salzlösungen mittels Mineralbasen erhalten.

Bevorzugt werden die im erfindungsgemäßen Verfahren bevorzugt verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze des eingesetzten Metalls/der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäß verwendetenen Katalysatoren weiterverarbeitet. Nach dem Waschen werden sie im bevorzugt bei 80 bis 200°C, vorzugsweise bei 100 bis 150°C, getrocknet und danach calciniert. Die Calcinierung wird bevorzugt bei Temperaturen zwischen 300 und 800°C, vorzugsweise 400 bis 600°C, insbesondere 450 bis 550°C, ausgeführt.

Nach der Calcinierung wird der Katalysator zweckmäßigerweise konditioniert, sei es, dass man ihn durch Vermahlen auf eine bestimmte Korngröße einstellt und/oder dass man ihn nach seiner Vermahlung mit Formhilfsmitteln wie Graphit oder Stearinsäure vermischt, mittels einer Presse zu den Formlingen, nämlich Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren können als solche gelagert werden. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200°C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400°C in einer Wasserstoffatmosphäre behandelt. Bei dieser Vorreduktion wird ein Teil der in den Katalysatoren vorliegenden sauerstoffhaltigen Metallverbindung/en zu dem/den entsprechenden Metall/en reduziert, so dass diese gemeinsam mit den verschiedenartigen Sauerstoffverbindungen in der aktiven Form des Katalysators vorliegen.

Die Umsetzung gemäß erfindungsgemäßem Verfahren erfolgt bevorzugt in einem Rohrreaktor.

Bei einer Monostranganlage besteht der Rohrreaktor, in dem die bevorzugt isotherme Umsetzung erfolgt, aus einer Hintereinanderschaltung mehrerer (z. B. zweier oder dreier) einzelner Rohrreaktoren.

Die bevorzugt isotherme Umsetzung gemäß dem erfindungsgemäßen Verfahren erfolgt bevorzugt mit einer Temperaturabweichung von maximal +/- 8 °C, besonders maximal +/- 5 °C, insbesondere maximal +/- 4 °C, ganz besonders maximal +/- 3 °C, z. B. maximal +/- 0 bis +/- 2 °C oder maximal +/- 0 bis +/- 1 °C.

Diese Temperaturabweichungen beziehen sich auf die jeweiligen Temperaturen im jeweiligen Katalysatorbett, und zwar beim Eintritt der Edukte in das Katalysatorbett und beim Austritt der Reaktionsmischung aus dem Katalysatorbett.

Dabei können mehrere Katalysatorbetten parallel oder in Reihe ge- oder verschaltet sein.

Sind mehrere Katalysatorbetten in Reihe geschaltet, beziehen sich die genannten Temperaturabweichungen bei der erfindungsgemäß bevorzugten isothermen Fahrweise auf die jeweilige Temperatur im Katalysatorbett, und zwar beim Eintritt der Edukte in das erste Katalysatorbett und beim Austritt der Reaktionsmischung aus dem letzten Katalysatorbett.

In einer bevorzugten Ausführungsform erfolgt die Temperierung des Reaktorrohrs von außen mit einem Wärmeträgerstrom, wobei es sich bei dem Wärmeträger z. B. um ein Öl, eine Salzschmelze oder eine andere wärmeübertragende Flüssigkeit handeln kann.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Der nach Kühlung und Entspannung anfallende flüssige Reaktionsaustrag wird gegebenenfalls von suspendierten Katalysatoren abgetrennt. Der katalysatorfreie Reaktionsaustrag enthält neben dem Zielprodukt, dem tertiären Methylamin I, noch überschüssigen Alkohol und gebildetes Wasser. Je nach Zahl der Methylierungsschritte entstehen zwei bis drei Mole Wasser, abhängig vom und bezogen auf eingesetzten Aminoalkohol (II, IIa). Außerdem findet man gegebenenfalls nicht umgesetzten Aminoalkohol, Zwischenprodukte, die nicht vollständig alkyliert wurden und gegebenenfalls geringe Mengen an Aminoalkohol-Nebenprodukten.

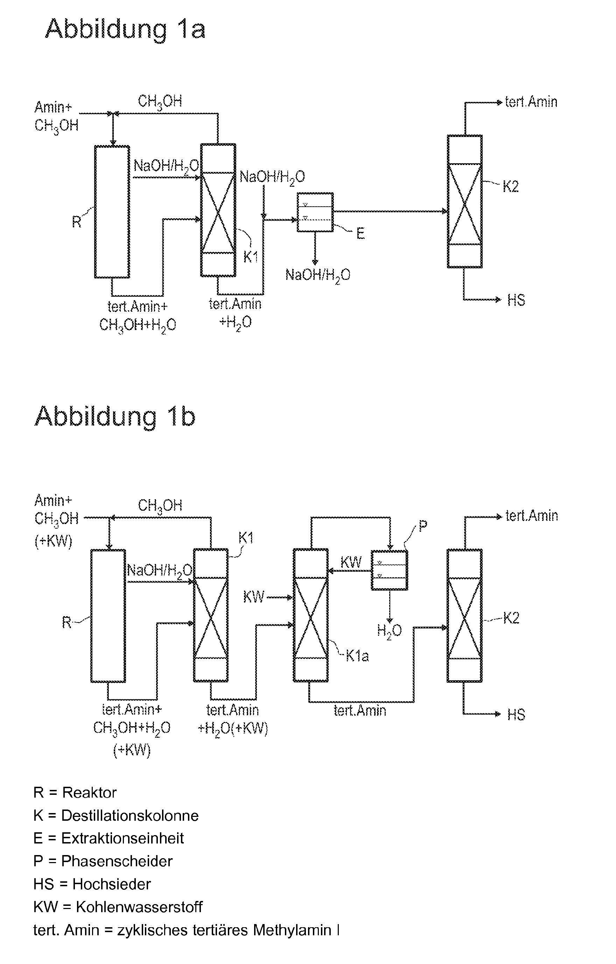

Die Aufarbeitung erfolgt insbesondere durch Destillation (vgl. Abb. 1 a und 1 b für bevorzugte Ausführungsformen).

Erfindungsgemäß wurde auch erkannt:

Wurde aus dem Reaktionsaustrag, z. B. in einer ersten Destillationskolonne K 1, überschüssiges Methanol über Kopf abdestilliert und mit frischem Aminoalkohol (II, IIa) gemischt, kam es ohne Vorheizen der beiden Ströme bzw. des gemeinsamen Stromes bei einer Temperatur < 50 °C, besonders < 40 °C, in dem Zuleitungsrohr zum Reaktor und an der Einstrittsstelle in den Reaktor zu Verkrustungen und Verstopfungen durch Feststoffe. Da im Reaktionsaustrag Kohlendioxid nachgewiesen werden konnte, dürfte es sich bei den Feststoffen um Carbamate handeln.

Die Verkrustungen und Verstopfungen ließen sich dadurch verhindern, dass, z. B. am Kopf der Destillationskolonne K 1, eine wässrige Lösung von Alkalimetall- oder Erdalkalimetallhydroxiden zugeführt wurde.

In einer besonderen Verfahrensvariante fügt man demnach der ersten Destillationseinheit in Schritt (ii) eine wässrige Lösung eines Alkali- oder Erdalkalihydroxids zu.

Die Menge an Alkalimetall- bzw. Erdalkalimetallhydroxid beträgt bevorzugt 0,0001 bis 0,1 Mol pro Mol Methanol.

Die Wassermenge ist nicht kritisch, da diese zusammen mit dem Reaktionswasser abgetrennt werden kann. Bevorzugt sind wässrige Lösungen, die 20 bis 50 Gew.-% an Alkalimetall- oder Erdalkalimetallhydroxiden, bevorzugt Natriumhydroxid und Kaliumhydroxid, enthalten.

Wurde mit dieser Maßnahme Methanol, z. B. über Kopf der Kolonne K 1, zurückgeführt, mit frischem Aminoalkohol (II, IIa) gemischt, auf Temperaturen im Bereich von 130 bis 270 °C vorgeheizt und in den Reaktor geleitet, so trat keine Feststoffbildung auf.

Erfindungsgemäß wird bevorzugt in einem Schritt (ii) der Kohlendioxidgehalt des zurückgeführten Methanols abgesenkt, in einer Ausgestaltung bevorzugt dadurch, dass das Rück-Methanol in flüssiger Phase bei einer Temperatur im Bereich von 0 bis 230 °C, besonders 5 bis 170 °C, weiter besonders 10 bis 85 °C, mit einem Inertgas, wie N₂ oder Ar, gestrippt wird. Dieses Strippen erfolgt bevorzugt über eine Zeitraum von 0,1 Minuten bis 5 Stunden, besonders von 1 Minute bis 1 Stunde. Damit das Methanol in flüssiger Phase verbleibt muss bei höheren Temperaturen entsprechend ein Überdruck, z. B. 1 bis 100 bar, angewendet werden.

In einer anderen Ausgestaltung wird bevorzugt in Schritt (ii) der Kohlendioxidgehalt des zurückgeführten Methanols dadurch abgesenkt, dass das Rück-Methanol in flüssiger Phase auf eine Temperatur im Bereich von 20 bis 120 °C, besonders von 30 bis 65 °C, erwärmt wird. Diese Erwärmung erfolgt bevorzugt über eine Zeitraum von 1 Minute bis 24 Stunden, besonders von 1 Minute bis 12 Stunden. Damit das Methanol in flüssiger Phase verbleibt muss bei höheren Temperaturen entsprechend ein Überdruck, z. B. 1 bis 10 bar, angewendet werden.

Der Kohlendioxidgehalt des zurückgeführten Methanols wird bevorzugt soweit abgesenkt, dass in der Methanol-Zuführungsleitung zum Reaktor, im Reaktor und insbesondere der Eintrittsstelle des Methanols in den Reaktor keine Feststoffbildung erfolgt. Die Methanol-Zuführungsleitung zum Reaktor schließt die Leitung zu einem ggf. vorhandenen Vorheizer vor dem Reaktor mit ein.

Im Besonderen senkt man in Schritt (ii) den Kohlendioxidgehalt des zurückgeführten Methanols auf einen Wert im Bereich von 0 bis 15 mol%, insbesondere von 0,001 bis 5 mol%, (jeweils bezüglich Methanol), ab.

Bevorzugt werden bei kontinuierlicher Fahrweise Aminoalkohol (II, IIa) und Rück-Methanol aus Schritt (ii), statt in einem gemeinsamen Strom, in zwei getrennten Strömen in den Reaktor des Reaktionsschritts (i) geleitet.

Insbesondere werden das Rück-Methanol aus Schritt (ii) und der Aminoalkohol vor dem Einbringen in den Reaktor des Reaktionsschritts (i) jeweils oder als Gemisch auf eine Temperatur im Bereich von 20 bis 240 °C, besonders 40 bis 150 °C, weiter besonders 50 bis 100 °C, vorgeheizt.

Besonders bevorzugt ist eine Kombination von Vorheizen, insbesondere des Rück-Methanols, mit der Absenkung des Kohlendioxidgehalts.

Das Sumpfprodukt der Methanolabdestillation, z. B. der Kolonne K 1, kann zur Abtrennung des Reaktionswassers mit 20 bis 50 Gew.-%iger wässriger Alkali- oder Erdalkalihydroxid-Lösung extrahiert werden. Die wässrige Hydroxidlösung wird durch Phasentrennung abgetrennt und ausgeschleust, die organische Phase bevorzugt in einer weiteren Destillationseinheit, z. B. Kolonne K 2, so destilliert, dass als Kopfprodukt das tertiäre Amin I, als Sumpfprodukt Hochsieder (HS) erhalten werden (vgl. Abb. 1 a).

Diese Entwässerungsmethode (vgl. Abb. 1 a) besitzt den Vorteil, dass das als Destillationssumpf, z. B. am Sumpf der Kolonne K 1, anfallende Gemisch aus Alkalimetall- und Erdalkalimetallcarbonat und Alkalimetall- und/oder Erdalkalimetallhydroxid zusammen mit der zur Entwässerung zugeführten wässrigen Hydroxidlösung abgetrennt oder ausgeschleust werden kann.

In einer weiteren bevorzugten Ausführungsform der Methylierung mit Methanol wird der Destillationssumpf nach der Methanol-Abtrennung, z. B. der Sumpfstrom aus der Kolonne K 1, der gegebenenfalls schon einen Kohlenwasserstoff (besonders C₄-C₁₀-Kohlenwasserstoff, wie Heptane, Oktane, Cyclohexane, Cycloheptane, als Lösungsmittel enthält, einer weiteren Destillationseinheit, z. B. Kolonne K 1a, zugeführt. Wenn notwendig wird in der weiteren Destillationseinheit, wie der Kolonne K 1a, eine weitere Menge des Kohlenwasserstoffs eingespeist. Durch Azeotropdestillation wird ein zweiphasiges Kohlenwasserstoff/Wasser-Azeotrop über Kopf abdestilliert. Im Phasenscheider P wird die Wasserphase ausgeschleust und die Kohlenwasserstoffphase auf den Kopf der Kolonne zurückgegeben. Der Sumpfstrom der Kolonne K 2 wird, z. B. wie in Abb. 1 a gezeigt, zur Gewinnung von reinem tertiären Amin I einer weiteren Destillationseinheit, z. B. der Kolonne K 2, zugeführt (vgl. Abb. 1 b).

Mit dem erfindungsgemäßen Verfahren herstellbar sind tertiäre Amine der Formel I 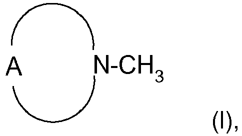 in der
A eine C₄-Alkylengruppe, eine C₅-Alkylengruppe oder eine -(CH₂)₂-B-(CH₂)₂-Gruppe bedeutet, wobei B Sauerstoff (O) oder einen Rest N-R¹ bedeutet und R¹ C₁- bis C₅-Alkyl, Aryl oder C₅- bis C₇-Cycloalkyl bedeutet,
durch Umsetzung eines Aminoalkohols II aus der Gruppe 1,4-Aminobutanol, 1,5-Aminopentanol, Aminodiglykol (ADG) bzw. Aminoethyl-ethanolamin der Formel IIa 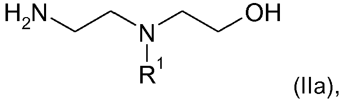 in der R¹ wie oben genannnt bedeutet oder auch Wasserstoff (H) bedeutet, wobei in I dann R¹ = CH₃ ist, mit Methanol.

Bevorzugt ist die Herstellung von N-Methyl-pyrrolidin durch Umsetzung von 1,4-Aminobutanol.

Auch bevorzugt ist die Herstellung von N-Methyl-piperidin durch Umsetzung von 1,5-Aminopentanol.

Auch bevorzugt ist die Herstellung von N-Methyl-morpholin durch Umsetzung von Aminodiglykol (ADG).

Weiter bevorzugt ist die Herstellung von N,N'-Dimethyl-piperazin durch Umsetzung von Aminoethyl-ethanolamin der Formel IIa 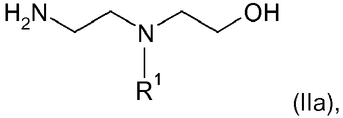 in der R¹ Wasserstoff (H) oder Methyl bedeutet.

Der Substituent R¹ in den Verbindungen I bzw. IIa hat folgende Bedeutung:
R¹:
   - C₁₋₅-Alkyl, bevorzugt C₁₋₃-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl,
   - C₅₋₇-Cycloalkyl, bevorzugt C₅₋₆-Cycloalkyl, wie Cyclopentyl, Cyclohexyl, Cycloheptyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - in IIa auch Wasserstoff (H), wobei im Umsetzungsprodukt I dann R¹ = CH₃ darstellt.

Die Synthese der Amine I kann als zyklisierende Methylierung bezeichnet werden. Bei der zyklisierenden Methylierung von 1,4-Aminobutanol können die Zwischenstufen 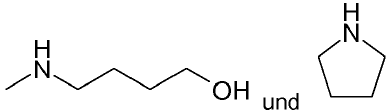 durchlaufen werden.

Bei der zyklisierenden Methylierung von 1,5-Aminopentanol können die Zwischenstufen 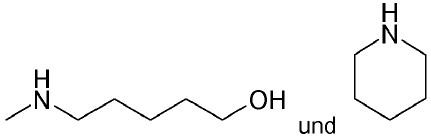 durchlaufen werden.

Bei der zyklisierenden Methylierung von Aminodiglykol (H₂N(CH₂)₂O(CH₂)₂OH, ADG)
können die Zwischenstufen 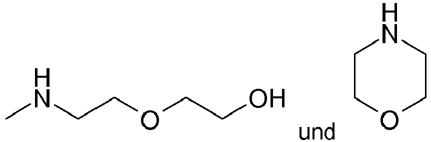 durchlaufen werden.

Bei der zyklisierenden Methylierung von Aminoethyl-ethanolamin der Formel IIa mit R¹ = H können die Zwischenstufen 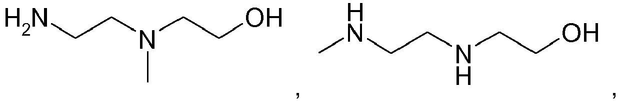 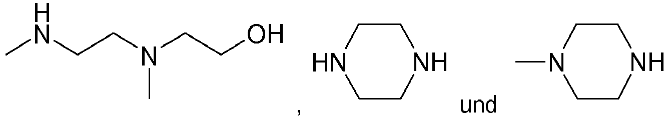 durchlaufen werden.

Bei der zyklisierenden Methylierung von Aminoethyl-ethanolamin der Formel IIa mit R¹
= CH₃ können die Zwischenstufen 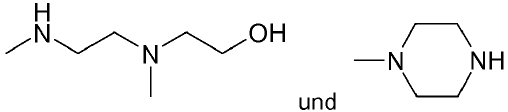 durchlaufen werden.

Alle Druckangaben beziehen sich auf den Absolutdruck.

### Beispiele

Für die folgenden Beispiele wurde ein Kupfer-Katalysator der Zusammensetzung 55 Gew.-% CuO und 45 Gew.-% gamma-Al₂O₃ (nach dessen letzter Wärmebehandlung und vor dessen Reduktion mit Wasserstoff) verwendet (Katalysator A).

Die Herstellung des Katalysators erfolgte durch Tränkung von gamma-Al₂O₃ - Pulver mit einer wässrigen Kupfernitrat-Lösung. Die Tablettierung erfolgte nach üblicher Methode. Vor Beginn der Umsetzung wurde der Katalysator im Wasserstoffstrom bei ca. 200°C reduziert (s.u.).

### Beispiel 1

Herstellung von N,N'-Dimethylpiperazin aus Aminoethylethanol und Methanol

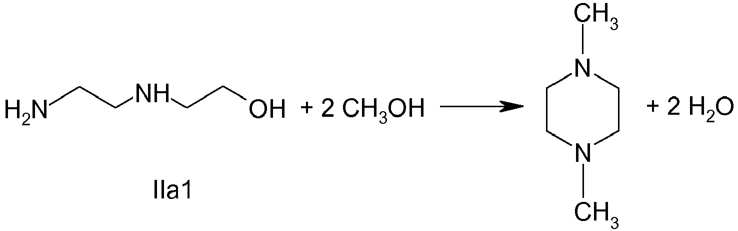

Die Umsetzung wurde in einem magnetgekuppelten 300 ml Rührautoklaven mit Elektroheizung und Innentemperatur-Kaskadenregelung durchgeführt.

In den mit Stickstoff inertisierten Autoklaven wurden 15,6 g Aminoethylethanolamin (IIa1) (0,15 mol), 102,1 g Methanol (3,2 mol) und 15 g eines reduzierten und passivierten Kupfer auf Aluminiumoxid enthaltenden Katalysators A (3 x 3 mm Tabletten) eingefüllt. Der Katalysator bestand vor der Reduktion zu 55 Gew.-% aus Kupferoxid (CuO) und zu 45 Gew.-% aus Aluminiumoxid. Die Reduktion war vor der Umsetzung bei 180 bis 200 °C, die Passivierung bei < 50 °C mit Luft erfolgt. Auf das Reaktionsgemisch wurde bei Raumptemperatur Wasserstoff bis zu einem Druck von 10 bar aufgepresst. Dann wurde auf 200 °C erhitzt, Wasserstoff bis zu einem Gesamtdruck von 80 bar nachgepresst und 12 Stunden lang bei 200 °C und 80 bar gerührt (800 rpm).

Die gaschromatographische Analyse (GC-Säule 30 m RTX 5 Amin) ergab, dass der Reaktionsaustrag (ohne überschüssiges Methanol) bei vollständigem Aminoethyl-ethanolamin-Umsatz zu 88,5 Flächen-% aus N,N'-Dimethylpiperazin bestand.

Aus Beispiel 2 in EP 257 443 B1 ist bekannt, das primäre Amin n-Pentylamin in Gegenwart von Kupfer-Katalysatoren mit dem primären Alkohol n-Pentanol zu dem tertiären Amin Tri-n-Pentylamin umzusetzen. Daher hätte man erwartet, dass Aminoethylethanolamin IIa1 sich ganz oder zumindest teilweise zu N,N'-Dimethylaminoethyl-ethanolamin III mit R¹ = H und/oder dessen Methylierungsprodukt III mit R¹ = CH₃ umsetzen würde.

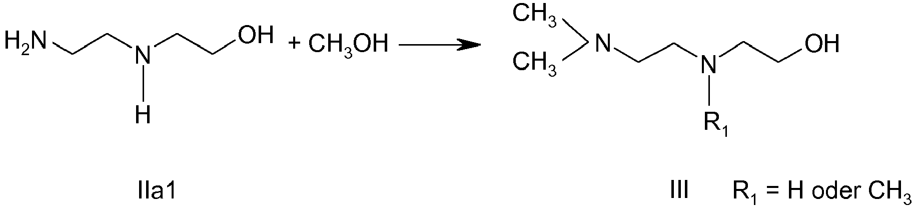

Beide Verbindungen III können keine Cyclisierung zu N,N'-Dimethylpiperazin eingehen. III wurde gaschromatographisch nicht gefunden. Dieses Ergebnis und die hohen Ausbeuten an N,N'-Dimethylpiperazin sind daher für den Fachmann überraschend.

Die gleiche Begründung für ein überraschendes Ergebnis gilt für andere Ausgangsverbindungen II, wie 1,4-Aminobutanol, 1,5-Aminopentanol und Aminodiglykol (ADG).

### Beispiel 2

Herstellung von N-Methylmorpholin aus Aminodiglykol und Methanol

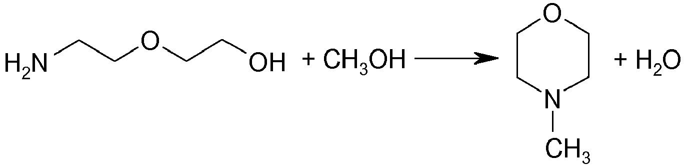

Beispiel 2 wurde in der in Beispiel 1 beschriebenen Apparatur durchgeführt. In den mit Stickstoff inertisierten Autoklaven wurden 15,7 g Aminodiglykol (0,15 mol), 96 g Methanol (3 mol) und 10 g des in Beispiel 1 beschriebenen, reduzierten und passivierten Katalysators (A) eingefüllt. Auf das Reaktionsgemisch wurden bei Raumtemperatur 10 bar Wasserstoff aufgepresst. Dann wurde auf 180 °C erwärmt und Wasserstoff bis zu einem Druck von 80 bar nachgepresst. Das Reaktionsgemisch wurde 6 Stunden lang bei 180 °C und 80 bar gerührt (700 rpm). Dann wurde der Autoklav abgekühlt und entspannt. Die gaschromatographische Analyse (GC-Säule und Analysenbedingungen wie in Beispiel 1) ergab, dass der Reakionsaustrag bei vollständigem Umsatz des Aminodiglykols zu 85,5 Flächen% aus N-Methylmorpholin bestand.

### Beispiel 3

Herstellung von N-Methylpiperidin aus 5-Aminopentanol und Methanol

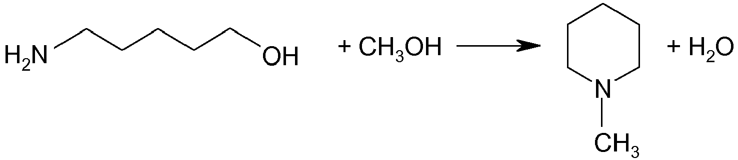

Beispiel 3 wurde in der in Beispiel 1 beschriebenen Apparatur durchgeführt. In den mit Stickstoff inertisierten Autoklaven wurden 31 g 5-Aminopentanol (0,3 mol), 102 g Methanol (3,2 mol) und 3 g des in Beispiel 1 beschriebenen, reduzierten und passivierten Katalysators (A) eingefüllt. Auf das Reaktionsgemisch wurden bei Raumtemperatur 5 bar Wasserstoff aufgepresst. Dann wurde auf 200 °C erwärmt und Wasserstoff bis zu einem Druck von 80 bar nachgepresst. Das Reaktionsgemisch wurde 12 Stunden bei 200 °C und 80 bar gerührt (800 rpm). Dann wurde der Autoklav abgekühlt und entspannt. Die gaschromatographische Analyse (GC-Säule und Analysenbedingungen wie in Beispiel 1) ergab, dass der Reaktionsaustrag bei vollständigem Umsatz des 5-Aminopentanols zu 88 Flächen% aus N-Methylpiperidin bestand.

## Patentansprüche

1. Verfahren zur Herstellung eines zyklischen tertiären Methylamins der Formel I 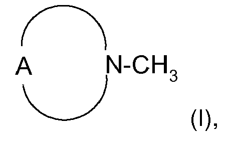 in der A eine C₄-Alkylengruppe, eine C₅-Alkylengruppe oder eine -(CH₂)₂-B-(CH₂)₂-Gruppe bedeutet, wobei B Sauerstoff (O) oder einen Rest N-R¹ bedeutet und R¹ C₁- bis C₅-Alkyl, Aryl oder C₅- bis C₇-Cycloalkyl bedeutet,
**dadurch gekennzeichnet, dass** man
(i) einen Aminoalkohol II aus der Gruppe 1,4-Aminobutanol, 1,5-Aminopentanol, Aminodiglykol (ADG) bzw. Aminoethyl-ethanolamin der Formel IIa 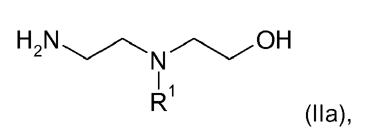
in der R¹ wie oben genannnt oder auch Wasserstoff (H) bedeutet, wobei im Amin I dann R¹ = CH₃ ist,
mit Methanol in einem Reaktor bei einer Temperatur im Bereich von 150 bis 270 °C in Gegenwart eines kupferhaltigen Heterogenkatalysators in der Flüssigphase umsetzt, wobei das Methanol in der 1- bis 25-fachen molaren Menge bezogen auf den eingesetzten Aminoalkohol (II, IIa) eingesetzt wird.

2. Verfahren nach Anspruch 1, **dadurch gekennzeichnet, dass** man
(ii) aus dem Reaktionsaustrag der Umsetzung (Reaktionsschritt (i)) in einer ersten Destillationseinheit unumgesetztes Methanol als Kopfprodukt abtrennt und in den Reaktionsschritt (i) zurückführt, wobei der Kohlendioxidgehalt des zurückgeführten Methanols soweit abgesenkt wird, dass in der Methanol-Zuführungsleitung zum Reaktor und der Eintrittsstelle des Methanols in den Reaktor keine Feststoffbildung erfolgt.

3. Verfahren nach Anspruch 2, **dadurch gekennzeichnet, dass** man
(iii) aus dem Sumpfprodukt der ersten Destillationseinheit durch Extraktion mit wässriger Alkali- oder Erdalkalihydroxid-Lösung oder durch Azeotropdestillation mit einem Kohlenwasserstoff in einer zweiten Destillationseinheit Reaktionswasser entfernt, und
(iv) den resultierenden Austrag zur Gewinnung von tertiärem Amin I fraktionierend destilliert.

4. Verfahren nach einem der beiden vorhergehenden Ansprüche, **dadurch gekennzeichnet, dass** in Schritt (ii) der Kohlendioxidgehalt des zurückgeführten Methanols dadurch abgesenkt wird, dass das Rück-Methanol in flüssiger Phase auf eine Temperatur im Bereich von 20 bis 120 °C erwärmt wird.

5. Verfahren nach den Ansprüchen 2 oder 3, **dadurch gekennzeichnet, dass** in Schritt (ii) der Kohlendioxidgehalt des zurückgeführten Methanols dadurch abgesenkt wird, dass das Rück-Methanol in flüssiger Phase bei einer Temperatur im Bereich von 0 bis 230 °C mit einem Inertgas gestrippt wird.

6. Verfahren nach einem der vier vorhergehenden Ansprüche, **dadurch gekennzeichnet, dass** in Schritt (ii) der Kohlendioxidgehalt des zurückgeführten Methanols auf einen Wert im Bereich von 0 bis 15 mol% abgesenkt wird.

7. Verfahren nach einem der fünf vorhergehenden Ansprüche, **dadurch gekennzeichnet, dass** man der ersten Destillationseinheit in Schritt (ii) eine wässrige Lösung eines Alkali- oder Erdalkalihydroxids zufügt.

8. Verfahren nach dem vorhergehenden Anspruch, **dadurch gekennzeichnet, dass** die Menge an Alkalimetall- bzw. Erdalkalimetallhydroxid 0,0001 bis 0,1 Mol pro Mol Methanol beträgt.

9. Verfahren nach einem der vorhergehenden Ansprüche, **dadurch gekennzeichnet, dass** die Umsetzung des Aminoalkohols (II, IIa) mit Methanol (Schritt (i)) kontinuierlich erfolgt.

10. Verfahren nach dem vorhergehenden Anspruch, **dadurch gekennzeichnet, dass** Aminoalkohol und Rück-Methanol aus Schritt (ii) in zwei getrennten Strömen oder gemeinsam in den Reaktor des Reaktionsschritts (i) geleitet werden.

11. Verfahren nach dem vorhergehenden Anspruch, **dadurch gekennzeichnet, dass** das Rück-Methanol aus Schritt (ii) und der Aminoalkohol vor dem Einbringen in den Reaktor des Reaktionsschritts (i) jeweils oder als Gemisch auf eine Temperatur im Bereich von 20 bis 240 °C vorgeheizt werden.

12. Verfahren nach einem der vorhergehenden Ansprüche zur Herstellung von N-Methyl-pyrrolidin durch Umsetzung von 1,4-Aminobutanol.

13. Verfahren nach einem der vorhergehenden Ansprüche 1 bis 11 zur Herstellung von N-Methyl-piperidin durch Umsetzung von 1,5-Aminopentanol.

14. Verfahren nach einem der vorhergehenden Ansprüche 1 bis 11 zur Herstellung von N-Methyl-morpholin durch Umsetzung von Aminodiglykol (ADG).

15. Verfahren nach einem der vorhergehenden Ansprüche 1 bis 11 zur Herstellung von N,N'-Dimethyl-piperazin durch Umsetzung von Aminoethyl-ethanolamin der Formel IIa 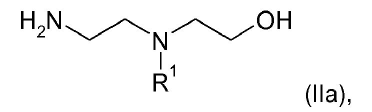 in der R¹ Wasserstoff (H) oder Methyl bedeutet.

16. Verfahren nach einem der vorhergehenden Ansprüche, **dadurch gekennzeichnet, dass** man die Umsetzung in Gegenwart eines kupfer- und aluminiumoxidhaltigen Katalysators durchführt.

17. Verfahren nach einem der vorhergehenden Ansprüche, **dadurch gekennzeichnet, dass** die katalytisch aktive Masse des Katalysators vor dessen Reduktion mit Wasserstoff 25 bis 80 Gew.-% Aluminiumoxid (Al₂O₃),
20 bis 75 Gew.-% sauerstoffhaltige Verbindungen des Kupfers, berechnet als CuO,
0 bis 2 Gew.-% sauerstoffhaltige Verbindungen des Natriums, berechnet als Na₂O, und weniger als 5 Gew.-% sauerstoffhaltige Verbindungen des Nickels, berechnet als NiO, enthält.

18. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Nickels, berechnet als NiO, enthält.

19. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Kobalts, berechnet als CoO, enthält.

20. Verfahren nach einem der vorhergehenden Ansprüche, **dadurch gekennzeichnet, dass** die katalytisch aktive Masse des Katalysators vor dessen Reduktion mit Wasserstoff
30 bis 70 Gew.-% Aluminiumoxid (Al₂O₃) und
30 bis 70 Gew.-% sauerstoffhaltige Verbindungen des Kupfers, berechnet als CuO, enthält.

21. Verfahren nach einem der vorhergehenden Ansprüche, **dadurch gekennzeichnet, dass** die katalytisch aktive Masse des Katalysators vor dessen Reduktion mit Wasserstoff 0,05 bis 1 Gew.-% sauerstoffhaltige Verbindungen des Natriums, berechnet als Na₂O, enthält.

22. Verfahren nach einem der vorhergehenden Ansprüche, **dadurch gekennzeichnet, dass** die katalytisch aktive Masse des Katalysators kein Nickel, Kobalt und/oder Ruthenium enthält.

23. Verfahren nach einem der vorhergehenden Ansprüche, **dadurch gekennzeichnet, dass** die Umsetzung isotherm, mit einer Temperaturabweichung von maximal +/- 8 °C erfolgt.

24. Verfahren nach einem der vorhergehenden Ansprüche, **dadurch gekennzeichnet, dass** man im Reaktionsschritt (i) dem Reaktor Wasserstoff (H₂) zuführt.

25. Verfahren nach einem der vorhergehenden Ansprüche 9 bis 24, **dadurch gekennzeichnet, dass** im Reaktionsschritt (i) die Umsetzung in einem Rohrreaktor erfolgt.

26. Verfahren nach einem der vorhergehenden Ansprüche 9 bis 24, **dadurch gekennzeichnet, dass** im Reaktionsschritt (i) die Umsetzung in einem Rohrbündelreaktor oder in einer Monostranganlage erfolgt.

27. Verfahren nach einem der vorhergehenden Ansprüche, **dadurch gekennzeichnet, dass** man im Reaktionsschritt (i) die Umsetzung bei einem Absolutdruck im Bereich von 50 bis 150 bar durchführt.

28. Verfahren nach einem der vorhergehenden Ansprüche, **dadurch gekennzeichnet, dass** der Katalysator im Reaktor als Festbett angeordnet ist.

## Claims

1. A process for preparing a cyclic tertiary methylamine of the formula I 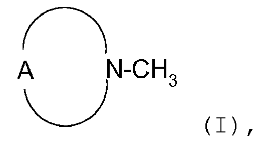 where
A is a C₄-alkylene group, a C₅-alkylene group or a -(CH₂)₂-B-(CH₂)₂- group, where B is oxygen (0) or an N-R¹ radical and R¹ is C₁-C₅-alkyl, aryl or C₅-C₇-cycloalkyl,
wherein
(i) an amino alcohol II from the group consisting of 1,4-aminobutanol, 1,5-aminopentanol, aminodiglycol (ADG) or aminoethylethanolamine of the formula IIa 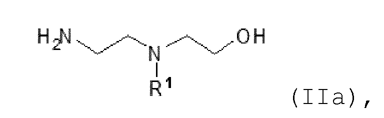
where R¹ is as defined above or is hydrogen (H), in which case R¹ = CH₃ in the amine I,
is reacted with methanol in a reactor at a temperature in the range from 150 to 270°C in the liquid phase in the presence of a copper-comprising heterogeneous catalyst, where the methanol is used in a from 1- to 25-fold molar amount based on the amino alcohol (II, IIa) used.

2. The process according to claim 1, wherein
(ii) unreacted methanol is separated off as overhead product from the reaction product mixture from the reaction (reaction step (i)) in a first distillation unit and recirculated to the reaction step (i), with the carbon dioxide content of the recirculated methanol being reduced to such an extent that no solids formation occurs in the methanol feed line to the reactor and the point of entry of the methanol into the reactor.

3. The process according to claim 2, wherein
(iii) water of reaction is removed from the bottom product from the first distillation unit by extraction with aqueous alkali metal hydroxide or alkaline earth metal hydroxide solution or by azeotropic distillation using a hydrocarbon in a second distillation unit and
(iv) the resulting output is fractionally distilled to obtain tertiary amine I.

4. The process according to either of the two preceding claims, wherein, in step (ii), the carbon dioxide content of the recirculated methanol is reduced by heating the recycle methanol in the liquid phase to a temperature in the range from 20 to 120°C.

5. The process according to claim 2 or 3, wherein, in step (ii), the carbon dioxide content of the recirculated methanol is reduced by stripping the recycle methanol in the liquid phase with an inert gas at a temperature in the range from 0 to 230°C.

6. The process according to any of the four preceding claims, wherein, in step (ii), the carbon dioxide content of the recirculated methanol is reduced to a value in the range from 0 to 15 mol%.

7. The process according to any of the five preceding claims, wherein an aqueous solution of an alkali metal hydroxide or alkaline earth metal hydroxide is introduced into the first distillation unit in step (ii).

8. The process according to the preceding claim, wherein the amount of alkali metal or alkaline earth metal hydroxide is from 0.0001 to 0.1 mol per mol of methanol.

9. The process according to any of the preceding claims, wherein the reaction of the amino alcohol (II, IIa) with methanol (step (i)) is carried out continuously.

10. The process according to the preceding claim, wherein amino alcohol and recycle methanol from step (ii) are introduced in two separate streams or together into the reactor of reaction step (i).

11. The process according to the preceding claim, wherein the recycle methanol from step (ii) and the amino alcohol are preheated individually or as a mixture to a temperature in the range from 20 to 240°C before introduction into the reactor of reaction step (i).

12. The process according to any of the preceding claims for the preparation of N-methyl-pyrrolidine by reaction of 1,4-aminobutanol.

13. The process according to any of the preceding claims 1 to 11 for the preparation of N-methylpiperidine by reaction of 1,5-aminopentanol.

14. The process according to any of the preceding claims 1 to 11 for the preparation of N-methylmorpholine by reaction of aminodiglycol (ADG).

15. The process according to any of the preceding claims 1 to 11 for the preparation of N,N'-dimethylpiperazine by reaction of aminoethylethanolamine of the formula IIa 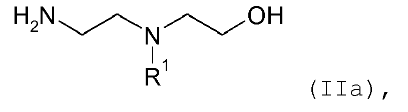 where R¹ is hydrogen (H) or methyl.

16. The process according to any of the preceding claims, wherein the reaction is carried out in the presence of a copper- and aluminum oxide-comprising catalyst.

17. The process according to any of the preceding claims, wherein the catalytically active composition of the catalyst before reduction with hydrogen comprises
from 25 to 80% by weight of aluminum oxide (Al₂O₃), from 20 to 75% by weight of oxygen-comprising compounds of copper, calculated as CuO,
from 0 to 2% by weight of oxygen-comprising compounds of sodium, calculated as Na₂O, and
less than 5% by weight of oxygen-comprising compounds of nickel, calculated as NiO.

18. The process according to any of the preceding claims, wherein the catalytically active composition of the catalyst before reduction with hydrogen comprises less than 1% by weight of oxygen-comprising compounds of nickel, calculated as NiO.

19. The process according to any of the preceding claims, wherein the catalytically active composition of the catalyst before reduction with hydrogen comprises less than 1% by weight of oxygen-comprising compounds of cobalt, calculated as CoO.

20. The process according to any of the preceding claims, wherein the catalytically active composition of the catalyst before reduction with hydrogen comprises
from 30 to 70% by weight of aluminum oxide (Al₂O₃) and
from 30 to 70% by weight of oxygen-comprising compounds of copper, calculated as CuO.

21. The process according to any of the preceding claims, wherein the catalytically active composition of the catalyst before reduction with hydrogen comprises from 0.05 to 1% by weight of oxygen-comprising compounds of sodium, calculated as Na₂O.

22. The process according to any of the preceding claims, wherein the catalytically active composition of the catalyst does not comprise any nickel, cobalt and/or ruthenium.

23. The process according to any of the preceding claims, wherein the reaction is carried out isothermally with a temperature deviation of not more than +/- 8°C.

24. The process according to any of the preceding claims, wherein hydrogen (H₂) is fed into the reactor in reaction step (i).

25. The process according to any of the preceding claims 9 to 24, wherein the reaction in reaction step (i) is carried out in a tube reactor.

26. The process according to any of the preceding claims 9 to 24, wherein the reaction in reaction step (i) is carried out in a shell-and-tube reactor or in a single line plant.

27. The process according to any of the preceding claims, wherein the reaction in reaction step (i) is carried out at an absolute pressure in the range from 50 to 150 bar.

28. The process according to any of the preceding claims, wherein the catalyst is arranged as a fixed bed in the reactor.

## Revendications

1. Procédé pour la préparation d'une méthylamine cyclique tertiaire de formule I 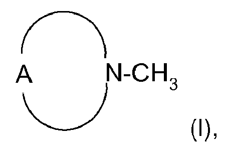 dans laquelle
A signifie un groupe C₄-alkylène, un groupe C₅-alkylène ou un groupe - (CH₂)₂-B-(CH₂)₂-, B signifiant oxygène (0) ou un radical N-R¹ et R¹ signifiant C₁-C₅-alkyle, aryle ou C₅-C₇-cycloalkyle, **caractérisé en ce qu'**on transforme
(i) un aminoalcool II du groupe formé par le 1,4-aminobutanol, le 1,5-aminopentanol, l'aminodiglycol (ADG) ou l'aminoéthyléthanolamine de formule IIa 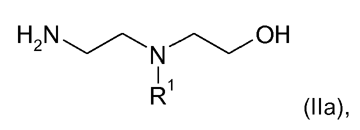 dans laquelle R¹ a la signification telle que mentionnée ci-dessus ou signifie également hydrogène (H), R¹ représentant alors CH₃ dans l'amine I,
avec du méthanol dans un réacteur à une température dans la plage de 150 à 270°C en présence d'un catalyseur hétérogène contenant du cuivre en phase liquide, le méthanol étant utilisé en 1 à 25 fois la quantité molaire par rapport à l'aminoalcool (II, IIa) utilisé.

2. Procédé selon la revendication 1, **caractérisé en ce qu'**on
(ii) sépare du produit de réaction de la transformation (étape de réaction (i)), dans une première unité de distillation, le méthanol non transformé comme produit de tête et on le recycle dans l'étape de réaction (i), la teneur en dioxyde de carbone du méthanol recyclé étant abaissée à un niveau tel qui ne se produit pas de formation de solide dans la conduite d'alimentation en méthanol vers le réacteur ni au niveau du site d'entrée du méthanol dans le réacteur.

3. Procédé selon la revendication 2, **caractérisé en ce qu'**on
(iii) élimine l'eau de réaction du produit du fond de la première unité de distillation, par extraction avec une solution aqueuse d'hydroxyde de métal alcalin ou d'hydroxyde de métal alcalino-terreux ou par distillation azéotropique avec un hydrocarbure, dans une deuxième unité de distillation, et
(iv) distille par fractionnement le produit résultant pour obtenir l'amine tertiaire I.

4. Procédé selon l'une quelconque des deux revendications précédentes, **caractérisé en ce que**, dans l'étape (ii), la teneur en dioxyde de carbone du méthanol recyclé est abaissée **en ce que** le méthanol recyclé en phase liquide est chauffé à une température dans la plage de 20 à 120°C.

5. Procédé selon les revendications 2 ou 3, **caractérisé en ce que**, dans l'étape (ii), la teneur en dioxyde de carbone du méthanol recyclé est abaissée **en ce que** le méthanol recyclé en phase liquide est épuisé par un gaz inerte à une température dans la plage de 0 à 230°C.

6. Procédé selon l'une quelconque des quatre revendications précédentes, **caractérisé en ce que**, dans l'étape (ii), la teneur en dioxyde de carbone du méthanol recyclé est abaissée à une valeur dans la plage de 0 à 15% en mole.

7. Procédé selon l'une quelconque des cinq revendications précédentes, **caractérisé en ce qu'**on ajoute à la première unité de distillation dans l'étape (ii) une solution aqueuse d'un hydroxyde de métal alcalin ou alcalino-terreux.

8. Procédé selon la revendication précédente, **caractérisé en ce que** la quantité d'hydroxyde de métal alcalin ou alcalino-terreux est de 0,0001 à 0,1 mole par mole de méthanol.

9. Procédé selon l'une quelconque des revendications précédentes, **caractérisé en ce que** la transformation de l'aminoalcool (II, IIa) avec du méthanol (étape (i)) est réalisée en continu.

10. Procédé selon la revendication précédente, **caractérisé en ce que** l'aminoalcool et le méthanol recyclé de l'étape (ii) sont guidés en deux flux séparés ou ensemble dans le réacteur de l'étape de réaction (i).

11. Procédé selon la revendication précédente, **caractérisé en ce que** le méthanol recyclé de l'étape (ii) et l'aminoalcool sont préchauffés avant l'introduction dans le réacteur de l'étape de réaction (i) chacun séparément ou sous forme de mélange à une température dans la plage de 20 à 240°C.

12. Procédé selon l'une quelconque des revendications précédentes pour la préparation de N-méthylpyrrolidine par transformation de 1,4-aminobutanol.

13. Procédé selon l'une quelconque des revendications précédentes 1 à 11 pour la préparation de N-méthylpipéridine par transformation de 1,5-aminopentanol.

14. Procédé selon l'une quelconque des revendications précédentes 1 à 11 pour la préparation de N-méthylmorpholine par transformation d'aminodiglycol (ADG).

15. Procédé selon l'une quelconque des revendications précédentes 1 à 11 pour la préparation de N,N'-diméthylpipéridine par transformation d'aminoéthyléthanolamine de formule IIa 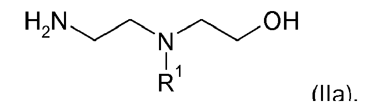 dans laquelle R¹ signifie hydrogène (H) ou méthyle.

16. Procédé selon l'une quelconque des revendications précédentes, **caractérisé en ce qu'**on réalise la transformation en présence d'un catalyseur contenant de l'oxyde de cuivre ou de l'oxyde d'aluminium.

17. Procédé selon l'une quelconque des revendications précédentes, **caractérisé en ce que** la masse catalytiquement active du catalyseur contient, avant sa réduction avec l'hydrogène
25 à 80% en poids d'oxyde d'aluminium (Al₂O₃),
20 à 75% en poids de composés oxygénés du cuivre, calculés sous forme de CuO,
0 à 2% en poids de composés oxygénés du sodium, calculés sous forme de Na₂O, et moins de 5% en poids de composés oxygénés du nickel, calculés sous forme de NiO.

18. Procédé selon l'une quelconque des revendications précédentes, **caractérisé en ce que** la masse catalytiquement active du catalyseur contient, avant sa réduction avec de l'hydrogène, moins de 1% en poids de composés oxygénés du nickel, calculés sous forme de NiO.

19. Procédé selon l'une quelconque des revendications précédentes, **caractérisé en ce que** la masse catalytiquement active du catalyseur contient, avant sa réduction avec de l'hydrogène, moins de 1% en poids de composés oxygénés du cobalt, calculés sous forme de CoO.

20. Procédé selon l'une quelconque des revendications précédentes, **caractérisé en ce que** la masse catalytiquement active du catalyseur contient, avant sa réduction avec l'hydrogène
30 à 70% en poids d'oxyde d'aluminium (Al₂O₃) et
30 à 70% en poids de composés oxygénés du cuivre, calculés sous forme de CuO.

21. Procédé selon l'une quelconque des revendications précédentes, **caractérisé en ce que** la masse catalytiquement active du catalyseur contient, avant sa réduction avec de l'hydrogène, 0,05 à 1% en poids de composés oxygénés du sodium, calculés sous forme de Na₂O.

22. Procédé selon l'une quelconque des revendications précédentes, **caractérisé en ce que** la masse catalytiquement active du catalyseur ne contient pas de nickel, de cobalt et/ou de ruthénium.

23. Procédé selon l'une quelconque des revendications précédentes, **caractérisé en ce que** la transformation a lieu de manière isotherme avec une déviation de la température d'au maximum ± 8°C.

24. Procédé selon l'une quelconque des revendications précédentes, **caractérisé en ce qu'**on alimente le réacteur en hydrogène (H₂) lors de l'étape de réaction (i).

25. Procédé selon l'une quelconque des revendications précédentes 9 à 24, **caractérisé en ce que**, dans l'étape de réaction (i), la transformation a lieu dans un réacteur tubulaire.

26. Procédé selon l'une quelconque des revendications précédentes 9 à 24, **caractérisé en ce que**, dans l'étape de réaction (i), la transformation a lieu dans un réacteur à faisceau tubulaire ou dans une installation en une seule ligne.

27. Procédé selon l'une quelconque des revendications précédentes, **caractérisé en ce que**, dans l'étape de réaction (i), on réalise la transformation à une pression absolue dans la plage de 50 à 150 bars.

28. Procédé selon l'une quelconque des revendications précédentes, **caractérisé en ce que** le catalyseur est disposé dans le réacteur sous forme de lit fixe.
